(19) Europäisches Patentamt
European Patent Office
Office européen des brevets

(11) Numéro de publication : **0 511 075 A1**

(12) # DEMANDE DE BREVET EUROPEEN

(21) Numéro de dépôt : **92401111.7**

(51) Int. Cl.$^5$ : **C08B 37/10,** A61K 31/725

(22) Date de dépôt : **21.04.92**

(30) Priorité : **23.04.91 FR 9104991**

(43) Date de publication de la demande :
**28.10.92 Bulletin 92/44**

(84) Etats contractants désignés :
**PT**

(71) Demandeur : **RHONE-POULENC RORER SA**
**20, avenue Raymond Aron**
**F-92160 Antony (FR)**

(72) Inventeur : **Baron, Jean-Pierre**
**17 rue Jules Ferry**
**F-77380 Combs la Viele (FR)**
Inventeur : **Brun, André**
**10 rue du 18 juin 1940**
**F-94700 Maisons Alfort (FR)**
Inventeur : **Hemker, Kendrik**
**Tongersestraat 1**
**NL-6211 LM Maastricht (Pays-Bas) (NL)**
Inventeur : **Uzan, André**
**35 avenue Victor Hugo**
**F-75116 Paris (FR)**

(74) Mandataire : **Savina, Jacques et al**
**Rhône-Poulenc Rorer S.A. Direction Brevets**
**(t144) 20 avenue Raymond Aron**
**F-92165 Antony Cédex (FR)**

(54) **Polysaccharides sulfates, procédé de préparation, composition pharmaceutique et utilisation.**

(57) L'invention concerne de nouveaux mélanges d'oligosaccharides sulfatés présentant la structure générale des oligosaccharides constitutifs de l'héparine, ayant une masse moléculaire moyenne de 6 ± 0,6 kD, une polydispersité proche de 1, et la capacité d'inhiber la génération de thrombine. L'invention concerne également leur préparation et des compositions pharmaceutiques les contenant.

EP 0 511 075 A1

Jouve, 18, rue Saint-Denis, 75001 PARIS

La présente invention concerne le domaine des polysaccharides de bas poids moléculaires. Plus particulièrement, elle concerne des compositions oligosaccharidiques présentant d'excellentes propriétés pharmacologiques et antithrombotiques.

D'une manière générale, les traitements antithrombotiques font appel à deux grandes catégories d'agents, à savoir les agents anticoagulants et les agents antiplaquettaires.

Parmi les agents anticoagulants, les composés anti-vitamine K constituent une famille très importante. Ces composés étant actifs par voie orale, ils sont utilisés dans de nombreuses indications. Cependant, leur emploi est encore limité par certains inconvénients et notamment les risques d'hémorragies qu'ils occasionnent et la difficulté d'adapter la posologie à un traitement de longue durée.

Les héparines constituent la seconde catégorie d'agents anticoagulants. Ce sont des substances biologiques d'extraction de la famille des glycosaminoglycans composées d'oligosaccharides ayant des longueurs de chaînes et des degrés de sulfatation variables. Les héparines sont utilisées dans différents types de thromboses, notamment dans le traitement ou la prévention des thromboses veineuses, éventuellement associées à d'autres thérapeutiques.

L'inconvénient des héparines réside dans leur activité anticoagulante élevée qui peut engendrer des hémorragies, et dans leur sensibilité à certains facteurs sériques, tels pf4, qui impose l'utilisation de doses relativement importantes.

Par ailleurs, les héparines sont des produits très hétérogènes. Il est donc difficile d'évaluer leur mécanisme d'action, d'apprécier la contribution de chacune des composantes dans l'activité globale de l'héparine, et, de ce fait, d'augmenter l'activité antithrombotique sans augmenter les effets secondaires.

Une première solution aux inconvénients mentionnés plus haut a été apportée par les héparines de bas poids moléculaires. Ces héparines sont obtenues par fragmentation (dépolymérisation) des chaînes oligosaccharidiques au moyen d'agents chimiques ou enzymatiques. En particulier, la dépolymérisation a été décrite par traitement d'un ester d'héparine en présence d'une base forte (EP 40144). Elle peut également être réalisée par traitement d'héparine en présence d'acide nitreux, ou par action d'une héparinase (EP 64452). Ces différents procédés conduisent à des mélanges d'oligosaccharides ayant la structure générale des polysaccharides constitutifs de l'héparine, mais ayant un poids moléculaire moyen en poids inférieur. Plus particulièrement, les recherches se sont éssentiellement orientées vers des mélanges dérivés de l'héparine ayant des chaines oligosaccharidiques très courtes. Ainsi, le brevet EP 27089 indique que des mélanges d'oligosaccharides dérivés de l'héparine ne renfermant pas plus de 8 motifs saccharidiques possédent une activité spécifique antithrombotique supérieure à l'héparine. De la même manière, des hexasaccharides ont été préparés et leurs propriétés antithrombotiques étudiées (EP 64452). On peut encore citer les brevets plus récents EP 84 999 et EP 301 618 sur des polysaccharides tels que des hexa-, des penta- et des tetrasaccharides dérivés de l'héparine.

Cependant, les produits décrits jusqu'à aujourd'hui n'ont pas permis de résoudre de manière totalement satisfaisante les problèmes rencontrés avec les héparines. En particulier, la correllation entre la masse moléculaire moyenne des produits et leurs effets secondaires n'a pu être confirmée in vivo.

La demanderesse a maintenant montré qu'il est possible d'obtenir, à partir d'héparines natives ou dépolymérisées, des mélanges d'oligosaccharides ayant des propriétés antithrombines nettement améliorées, et par conséquent, de meilleures potentialités thérapeutiques.

De manière inattendue, la demanderesse a en effet montré qu'une importante partie de l'activité antithrombotique de l'héparine était contenue dans une fraction étroite et homogène.

La présente invention résulte plus particulièrement de l'identification de fractions d'héparine monodispersées et ayant une masse moléculaire moyenne proche de 6 kD, possédant une activité antithrombine élevée.

Ainsi qu'illustré dans les exemples, il est donc possible d'obtenir des mélanges possédant une activité antithrombotique particulièrement importante en calibrant la masse moléculaire et en réduisant la polydispersité.

Un objet de l'invention réside dans un mélange d'oligosaccharides sulfatés présentant la structure générale des oligosaccharides constitutifs de l'héparine, caractérisé en ce qu'il présente une masse moléculaire moyenne en poids de $6 \pm 0,6$ kD et une polydispersité proche de 1, et en ce qu'il possède la capacité d'inhiber la génération de thrombine.

La polydispersité correspond au rapport de la masse moléculaire moyenne du mélange sur son poids moléculaire moyen en nombre. Elle rend compte de l'homogénéité moléculaire du mélange. Plus cette valeur est proche de 1 et plus le mélange est homogène.

En plus de leurs propriétés antithrombine, les mélanges de l'invention possèdent des propriétés pharmacocinétiques particulièrement avantageuses.

Ainsi, par rapport à l'héparine native et à ses formes dépolymérisées, les mélanges de l'invention présentent une sensibilité moins forte aux facteurs sériques tels pf4, ce qui augmente leur potentialité thérapeutique.

D'autres avantages des mélanges de l'invention résident notamment dans la réduction de certains effets secondaires indésirables, tels que :

- l'effet thrombocytopénique. L'un des inconvénients des mélanges connus dérivés de l'héparine provient de la chute du nombre de plaquettes qu'ils peuvent occasionner. Cet effet indésirable est fortement diminué lorsque les mélanges de l'invention sont employés.
- les réactions immunogènes. Lorsque de telles réactions sont trop importantes, il est clair que l'efficacité thérapeutique des produits est réduite. La faible immunogénicité des mélanges de l'invention constitue une autre de leurs caractéristiques pharmacologiques très intéressantes.

Par ailleurs, d'autres avantages des mélanges de l'invention résident notamment dans leurs excellentes biodisponibilité et demi-vie plasmatique.

Les propriétés énoncées ci-dessus permettent une utilisation pharmacologique particulièrement performante, notamment dans la prophylaxie et le traitement des thromboses veineuses ou artérielles. En outre, elles devraient rendre possible l'utilisation de doses plus importantes in vivo sans accroitre les risques hémorragiques.

Dans un mode préféré, les mélanges de l'invention sont plus particulièrement des fractions d'héparine dépolymérisée.

Comme indiqué précédemment, l'héparine dépolymérisée peut être obtenue par toute technique chimique, enzymatique ou autre, connue de l'homme de l'art permettant de fragmenter les chaines oligosaccharidiques de l'héparine. En particulier, les procédés décrits dans les brevets EP 40144, EP 64452, EP 37319 ou EP 337327 conviennent pour l'invention.

Encore plus préférentiellement, les mélanges de l'invention sont constitués d'oligosaccharides ayant un acide 2-O-sulfo 4 enopyranosuronique à l'une de leurs extrêmités.

Un mélange particulièrement avantageux est constitué par une fraction d'héparine dépolymérisée par action d'une base sur un ester d'héparine.

L'activité antithrombine des mélanges de l'invention peut être démontrée dans un test où la génération de thrombine est déclenchée en présence de thromboplastine humaine (voie extrinsèque) ou par contact (voie intrinsèque). Un tel test a été décrit précedemment (Hemker et al., Thromb. Haemostas. 56, 9-17, 1986).

Cette activité peut être exprimée de manière quantitative, par la quantité de produit nécessaire à l'inhibition de 25 % de la génération de thrombine. De cette manière, l'accroissement d'activité des mélanges de l'invention apparait clairement puisque, in vitro, leur activité spécifique antithrombine est, d'une manière surprenante, augmentée d'un facteur supérieur à 100 % par rapport à l'héparine utilisée au départ. Compte tenu des propriétés pharmacocinétiques particulièrement avantageuses des mélanges de l'invention, cette augmentation d'activité spécifique est encore plus importante in vivo.

Plus particulièrement, les mélanges de l'invention permettent, dans un test effectué sur plasma pauvre en plaquettes, d'inhiber 25 % de la génération de thrombine à des concentrations inférieures à 300 ng/ml.

Un autre objet de l'invention concerne un procédé de préparation d'un mélange tel que défini ci-avant caractérisé en ce que l'on fractionne une héparine ou une héparine dépolymérisée par gel-filtration.

Le procédé de l'invention fait intervenir plusieurs paramètres, dont le contrôle permet de calibrer la masse moléculaire du mélange final, et de fixer sa polydispersité. Ces paramètres sont notamment la force ionique de l'éluant et la nature du support utilisé.

Plus préférentiellement, le fractionnement est caractérisé en ce que l'on réalise successivement les étapes consistant à (i) mettre l'héparine ou l'héparine dépolymérisée de départ en solution dans l'éluant, (ii) faire passer la solution ainsi obtenue à travers une colonne au moins contenant le support solide pour la gel-filtration préalablement équilibrée avec le même éluant, et (iii) récupérer les fractions de poids moléculaire désiré.

Dans un mode particulier de mise en oeuvre de l'invention, on préfère utiliser comme héparine de départ une héparine dépolymérisée.

Encore plus préférentiellement, on utilise une héparine dépolymérisée par action d'une base sur un ester d'héparine. En particulier, la dépolymérisation peut être réalisée en milieu aqueux ou au sein d'un solvant organique inerte, sous l'action d'une base organique ou minérale telle que par exemple d'hydroxyde de sodium ou de potassium, d'un carbonate alcalin ou d'une amine tertiaire (triéthylamine, triéthylène- diamine, etc). L'action de la base sur l'ester permet de réaliser une dépolymérisation partielle et contrôlée de l'héparine sans altérer sa structure générale.

Plus généralement, les conditions de dépolymérisation décrites dans le brevet EP 40144 sont utilisables dans la présente invention.

Comme éluant utilisable dans le procédé de l'invention, on peut citer différents types de solutions salines, telles que des solutions de chlorure de sodium. Cependant, la demanderesse a montré que, pour obtenir des fractions ayant les meilleures propriétés, il est particulièrement avantageux de réaliser le fractionnement en utilisant un éluant choisi parmi les tampons phosphate, tels que notamment phosphate de potassium, phosphate de sodium ou $NH_4H_2PO_4$. Il est encore possible d'utiliser des solutions $NaClO_4$ ou $NH_4NO_3$ qui permettent d'obtenir des mélanges ayant d'excellentes caractéristiques.

La concentration de l'éluant et donc sa force ionique sont adaptées au mélange final recherché. Notamment, la concentration de l'éluant est avantageusement inférieure à 1M, et, encore plus préférentiellement, comprise entre 0,1 et 0,5 M.

Lorsque l'on utilise un tampon phosphate, il est particulièrement avantageux d'opérer à des concentrations proches de 0,2 M.

Dans la seconde étape du procédé de l'invention, le support utilisé est généralement choisi en fonction de la masse moléculaire moyenne du mélange de départ (héparine native, dépolymérisée ..), du produit final recherché, et du comportement du mélange de départ dans l'éluant utilisé. Avantageusement, on utilise comme support un gel de type polyacrylamide-agarose. A titre d'exemple on peut citer les gels AcA 54, AcA 202, séphadex G25 ou G50, ou encore Biogel P30 qui donnent de bons résultats.

Dans un premier mode particulièrement avantageux de mise en oeuvre du procédé de l'invention, lors de la seconde étape du fractionnement, le support solide est réparti en plusieurs colonnes disposées en série. Cette variante de l'invention permet d'employer des quantités finales de support pour la gel-filtration importantes, sans les inconvénients de l'art antérieur, à savoir éssentiellement les phénomènes de tassement. De cette manière, la séparation est beaucoup plus nette, y compris dans les hauts poids moléculaires, en une seule opération de fractionnement, et les supports sont plus facilement régénérables.

Le nombre de colonnes utilisées est adapté par l'homme de l'art en fonction du volume et de la nature du gel employé, de manière à obtenir le meilleur équilibre entre l'efficacité de la séparation et l'effet néfaste dû au tassement du gel.

Pour des raisons pratiques de mise en oeuvre, on préfère généralement utiliser de la seconde étape du procédé un nombre de colonnes inférieur à 20.

A titre illustratif, 40 litres de gel AcA 202 peuvent être répartis en 10 colonnes de 4 litres.

Dans un autre mode particulièrement avantageux de mise en oeuvre du procédé de l'invention, on utilise lors de la seconde étape du fractionnement successivement 2 types de supports au moins, ayant des caractéristiques de séparation différentes. Cette variante de l'invention permet d'obtenir un fractionnement final de meilleure qualité.

A titre d'exemple, le fractionnement peut être réalisé sur la séquence suivante de gels : AcA 202 - AcA 54 - AcA 202.

Pour une meilleure mise en oeuvre de l'invention, il est important d'employer des quantités élevées de gel, de manière à réaliser une séparation plus nette et à obtenir une plus grande homogénéité. Compte tenu toutefois des débits assez lents utilisés pour ce genre de gel-filtration, le volume de gel doit être adapté à la quantité de produit à séparer, de manière à obtenir le meilleur équilibre entre la séparation et l'effet de diffusion longitudinale.

Avantageusement, dans le procédé de l'invention, le rapport héparine de départ (g)/ volume de gel (l) est inférieur à 2, et encore plus préférentiellement compris entre 0,5 et 1,5.

L'invention concerne également un procédé de préparation de mélanges d'oligosaccharides faiblement dispersés et de poids moléculaire calibré par fractionnement par gel-filtration sur support solide d'héparine ou d'héparine dépolymérisée, caractérisé en ce que le support solide est réparti en plusieurs colonnes disposée en série.

Un autre objet de l'invention concerne une composition pharmaceutique ayant comme principe actif un mélange tel que défini ci-avant. Une telle composition peut être utilisée de manière particulièrement avantageuse dans la prophylaxie ou le traitement ou la prévention des accidents thrombotiques. Plus précisément, elle peut être utilisée:

- dans la prévention des thromboses veineuses dans les situations à risques,
- dans la prévention des accidents thrombotiques artériels, notamment dans le cas d'infarctus du myocarde,
- en régime post-opératoire, dans la prévention de thromboses veineuses chez les malades chirurgicaux, ou encore,
- dans la prévention des thromboses dans le matériel chirurgical.

La présente invention sera plus complètement décrite à l'aide des exemples qui suivent, qui doivent être considérés comme illustratifs et non limitatifs.

Exemple 1 : Préparation de mélanges selon l'invention.

- Dépolymérisation de l'héparine

A une solution de 10 g d'héparinate de sodium dans 100 ml d'eau on ajoute une solution de 25 g de chlorwe de benzéthonium dans 125 ml d'eau. Le produit obtenu à température ambiante est filtré, lavé à l'eau puis séché. 15 g d'héparinate de benzéthonium ainsi obtenu sont mis en solution dans 75 ml de chlorwe de méthylène,

auxquels on ajoute 15 ml de chlorure de benzyle. La solution est chauffée à une température comprise entre 25 et 35°C pendant 25 heures. On ajoute alors 90 ml d'une solution à 10 % d'acétate de sodium dans le méthanol, filtre, lave au méthanol et sèche. 10 g d'ester benzylique de l'héparine obtenu dans les conditions décrites ci-dessus sous forme de sel de sodium sont dissous dans 250 ml d'eau. A cette solution chauffée à 60°C environ on ajoute 0,9 g de soude. La température est maintenue 1 heure 30 minutes à 60°C environ, et le mélange réactionnel est ensuite refroidi vers 20°C et neutralisé par addition d'acide chlorhydrique dilué. On ajuste alors la concentration du milieu à 10 % en chlorure de sodium, et le produit est précipité dans 750 ml de méthanol, filtré et séché.

- Plusieurs colonnes de verre sont utilisées pour le fractionnement :

(a) 1 colonne de diamètre 95 mm et de hauteur 2 m contenant 14 litres de gel AcA 202 (gel sous forme de billes de polyacrylamide-agarose, de diamètre compris entre 60 et 140 μm),

(b) 1 colonne de diamètre 50 mm et de hauteur 2 m contenant 4 litres de gel AcA 54 gel sous forme de billes de polyacrylamide-agarose, de diamètre compris entre 60 et 140 μm),

(c) 2 colonnes de diamètre 50 mm et de hauteur 1 m contenant 2 litres de gel AcA 202.

Une solution contenant 20 g d'héparine dépolymérisée dans les conditions décrites précédemment est placée en tête de la colonne (a) et éluée par une phase mobile constituée d'une solution 0,33M de NaCl, à un débit de 210 ml/heure.

Les fractions sont récoltées en fin de colonne (a) et chargées en tête de colonne (b). L'élution est effectuée avec la même solution, et les fractions recueillies sont passées successivement sur les 2 colonnes (c).

Ce traitement permet de séparer efficacement et de récupérer en sortie de colonne (c) une fraction ayant les caractéristiques suivantes:

Poids Moleculaire: 6100 +/- 200
Polydispersité : 1,01

Exemple 2:

- 10 colonnes de diamètre intérieur 2,5 cm et de hauteur 50 cm contenant chacune environ 0,25 litre de gel AcA 202 sont reliées en série,
- une solution contenant 2 g d'héparine dépolymérisée dans les conditions de l'exemple 1 est chargée en tête du dispositif et éluée par une solution aqueuse 0,2 M de KH2PO4 à un débit de 0,42 ml/min.
- à partir de 21 heures, 113 fractions de 12,6 ml sont recueillies.

Les caractéristiques de ces fractions sont données dans le tableau 1, dans lequel la masse moléculaire moyenne a été déterminée par réfractométrie.

Exemple 3 :

On procède comme dans l'exemple 2:
- 10 colonnes de diamètre intérieur 10 cm et de hauteur 50 cm contenant chacune 3 à 4 litres de gel AcA 202 sont reliées en série,
- une solution contenant 30 g d'héparine dépolymérisée dans les conditions de l'exemple 1 est chargée en tête du dispositif et éluée par une solution aqueuse 0,2 M de $KH_2PO_4$ à un débit de 6,8 ml/min.

On obtient des fractions ayant les caractéristiques de polydispersité souhaitées.

Exemple 4 :

L'activité antithrombine des mélanges de l'invention est mesurée sur du plasma stimulé par de la thromboplastine humaine (voie extrinsèque) ou par contact (phospholipides + kaoline : voie intrinsèque) dans les conditions décrites précédemment (Cf Hemker et al. précitée). L'activité est estimée par la diminution du pic de la courbe de génération de thrombine par rapport à un contrôle effectué en présence de tampon seulement. Les résultats sont exprimés par la CI25 : concentration nécessaire pour obtenir 25% d'inhibition de la génération de thrombine.

Protocole:

A un volume de plasma est ajouté 1/4 de volume de tampon Tris-HCl 50 mM, NaCl 0,1 M, pH 7,35 avec 0,5 mg/ml d'albumine bovine, contenant différentes concentrations d'échantillons à tester. Après une incubation de 5 min. à 37°C, la génération de thrombine est déclenchée par l'addition de 1/4 de volume de thromboplastine diluée 1:40 en CaCl2 0,1 M (système extrinsèque) ou par 6 μM de phospholipides (20 % phosphatidyl

sérine, 80 % phosphatidyl choline) et 0,15 mimi de kaoline en CaCl2 0,1 M (système intrinséque). La génération de thrombine est obtenue en mesurant à intervalles réguliers (15-30 sec.) l'activité amydolitique sur le substrat S2238, substrat chromogène à 405 nM spécifique pour la thrombine. Différentes concentrations des échantillons sont testées, afin d'obtenir 25 % d'ihnibition du contrôle.

Résultats :

Sur plasma pauvre en plaquettes

1 ) voie extrinsèque

Héparine dépolymérisée de départ : CI25 = 450 ng/ml
Mélange préparé dans l'exemple 1 : CI25 = 200 ng/ml

Gain d'activité : 125 %
2) voie intrinsèque

Héparine dépolymérisée de départ : CI25 = 550 ng/ml
Mélange préparé dans l'exemple 1 : CI25 = 250 ng/ml

gain d'activité : 120 %

Sur plasma riche en plaquettes :

Héparine dépolymérisée de départ : CI25 = 1100 ng/ml
Mélange préparé dans l'exemple 1 : CI25 = 500 ng/ml

Dans les mêmes conditions, l'héparine native (non dépolymérisée, non fractionnée) ne possède aucune activité inhibitrice à 2500 ng/ml.

TABLEAU 1

| N° FRACTION | MASSE MOLECULAIRE MOYENNE | POLYDISPERSITE |
|---|---|---|
| 14-17 | 10 712 | 1,027 |
| 18-20 | 8 400 | 1,013 |
| 21-22 | 7 519 | 1,010 |
| 23-24 | 6 986 | 1,011 |
| 26-27 | 6 365 | 1,008 |
| 28-31 | 5 874 | 1,009 |
| 32-35 | 5 295 | 1,011 |
| 36-40 | 4 761 | 1,012 |
| 42-46 | 4 192 | 1,013 |
| 48-53 | 3 608 | 1,016 |
| 56-61 | 2 988 | 1,019 |
| 64-70 | 2 359 | 1,023 |
| 75-80 | 1 758 | 1,029 |
| 83-85 | 1 476 | 1,028 |
| 88-94 | 1 176 | 1,027 |

**Revendications**

1. Mélange d'oligosaccharides sulfatés présentant la structure générale des oligosaccharides constitutifs de l'héparine caractérisé en ce qu'il présente une masse moléculaire moyenne de 6 ± 0,6 kD et une polydispersité proche de 1, et en ce qu'il possède la capacité d'inhiber la génération de thrombine.

2. Mélange selon la revendication 1 caractérisé en ce qu'il s'agit d'une fraction d'héparine dépolymérisée.

3. Mélange selon la revendication 2 caractérisé en ce qu'il est constitué d'oligosaccharides ayant un acide 2-O-sulfo 4 énopyranosuronique à l'une de leurs extrémités.

4. Mélange selon la revendication 3 caractérisé en ce qu'il s'agit d'une fraction d'héparine dépolymérisée par action d'une base sur un ester d'héparine.

5. Mélange selon l'une quelconque des revendications 1 à 4 caractérisé en ce que, dans un test sur plasma pauvre en plaquettes stimulé par de la thromboplastine humaine ou par contact, il possède la capacité d'inhiber 25 % de la génération de thrombine à des concentrations inférieures à 400 ng/ml.

6. Procédé de préparation d'un mélange selon l'une des revendications 1 à 4 caractérisé en ce que l'on fractionne une héparine ou une héparine dépolymérisée par gel-filtration.

7. Procédé selon la revendication 6 caractérisé en ce que le fractionnement comprend successivement les étapes consistant à (i) mettre l'héparine ou l'héparine dépolymérisée de départ en solution dans l'éluant, (ii) faire passer la solution ainsi obtenue à travers une colonne au moins contenant le support solide pour la gel-filtration, préalablement équilibrée avec le même éluant, et (iii) récupérer les fractions de poids moléculaire désiré.

8. Procédé selon la revendication 7 caractérisé en ce que l'on utilise une héparine dépolymérisée.

9. Procédé selon la revendication 8 caractérisé en ce que l'on utilise une héparine dépolymérisée par action d'une base sur un ester d'héparine.

10. Procédé selon la revendication 7 caractérisé en ce que l'éluant est constitué par un tampon phosphate, préférentiellement un tampon phosphate de sodium ou phosphate de potassium, ou par des solutions de $NaClO_4$ ou $NH_4NO_3$..

11. Procédé selon la revendication 7 caractérisé en ce que, lors de la seconde étape du fractionnement, le support est réparti en plusieurs colonnes disposées en série.

12. Procédé selon la revendication 11 caractérisé en ce que l'on utilise un nombre de colonnes inférieur à 20.

13. Procédé selon la revendication 7 caractérisé en ce que la seconde étape est réalisée en utilisant successivement 2 types de supports au moins, ayant des caractéristiques de séparation différentes.

14. Procédé selon l'une quelconque des revendications 7, 11, 12 et 13 caractérisé en ce que le support utilisé est un gel de type polyacrylamide-agarose.

15. Procédé selon la revendication 14 caractérisé en ce que le rapport héparine de départ (g)/ volume de gel (l) est inférieur à 2, et de préférence compris entre 0,5 et 1,5.

16. Procédé de préparation d'un mélange d'oligosaccharides faiblement dispersé et ayant un poids moléculaire calibré par fractionnement par gel-filtration sur support solide d'héparine ou d'héparine dépolymérisée, caractérisé en ce que le support solide est réparti en plusieurs colonnes diposées en série.

17. Composition pharmaceutique ayant comme principe actif un mélange d'oligosaccharides selon l'une quelconque des revendications 1 à 5.

18. Composition pharmaceutique selon la revendication 17 destinée au traitement et à la prévention des thromboses veineuses et artérielles.

19. Composition pharmaceutique selon la revendication 17 destinée à la prévention des accidents thrombotiques artériels, notamment dans le cas d'infarctus du myocarde,

20. Composition pharmaceutique selon la revendication 17 destinée à une utilisation en régime post-opératoire, dans la prévention de thromboses veineuses chez les malades chirurgicaux.

21. Utilisation d'un mélange d'oligosaccharides selon l'une quelconque des revendications 1 à 5 dans la prévention des thromboses dans le matériel chirurgical.

Office européen des brevets

# RAPPORT DE RECHERCHE EUROPEENNE

Numero de la demande

EP 92 40 1111

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | FR-A-2 548 672 (PHARMUKA LABORATORIES)<br>*Document en entier*<br>--- | 1-21 | C08B37/10<br>A61K31/725 |
| Y | DE-A-3 608 685 (SANDOZ-PATENT-GMBH)<br>* exemples 1,2 *<br>--- | 1-21 | |
| Y | EP-A-0 244 235 (NOVO INDUSTRI A/S)<br>* revendications 1,9 *<br>--- | 1-21 | |
| X | EP-A-0 337 327 (BIOIBERICA S.A.)<br>* page 6 - page 7 *<br>----- | 1-21 | |
| | | | **DOMAINES TECHNIQUES RECHERCHES (Int. Cl.5 )** |
| | | | C08B<br>A61K<br>C07H |

Le présent rapport a été établi pour toutes les revendications

| Lieu de la recherche | Date d'achèvement de la recherche | Examinateur |
|---|---|---|
| LA HAYE | 20 JUILLET 1992 | LENSEN H.W.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........
& : membre de la même famille, document correspondant

EPO FORM 1503 03.82 (P0402)